# EUROPÄISCHE PATENTSCHRIFT

(11) **EP 2 890 327 B1**
(45) Veröffentlichungstag und Bekanntmachung des Hinweises auf die Patenterteilung: **09.01.2019**
(21) Anmeldenummer: 13759418.0
(22) Anmeldetag: 30.08.2013
(51) Int. Cl.: A61C 7/12

(54) **MUNDEINLAGE**
ORAL INSERT
INSERT BUCCAL

(30) Priorität: 31.08.2012 CH 15602012
(43) Veröffentlichungstag der Anmeldung: 08.07.2015
(73) Patentinhaber: Steffling, Michael, 50000 Chiangmai (TH)
(72) Erfinder: Steffling, Michael, 50000 Chiangmai (TH)
(74) Vertreter: Riederer Hasler & Partner Patentanwälte AG
(86) Internationale Anmeldenummer: PCT/CH2013/000154
(87) Internationale Veröffentlichungsnummer: WO 2014/032196

(56) Entgegenhaltungen:
- WO-A1-00/35369
- WO-A1-95/19155

## Beschreibung

### Gebiet der Erfindung

Die Erfindung betrifft eine Mundeinlage gemäss Oberbegriff des Anspruchs 1.

### Stand der Technik

Das Tragen von Zahnspangen verursacht an den Lippeninnenseiten des Patienten Schmerzen. Dies liegt an den erhabenen und zumeist scharfkantigen Brackets an denen sich die empfindliche Mundschleimhaut reibt. Das Tragen von Zahnspangen und der dadurch notwendigerweise vorhandene Druck auf die Zähne führt zu Verspannungen der Mund und Gesichtsmuskulatur, wodurch ebenfalls Schmerzen entstehen.

Die US 5,447,168 zeigt einen Lippenschutz, der die Form eines gebogenen Streifens mit geringer Dicke hat. Der Streifen wird zwischen die Zähne und die Lippen auf der Zahnaussenseite platziert, um die Lippen gegen die Zahnaussenseite und alle dort befindlichen Elemente einer Zahnspange abzuschirmen. Die Länge ist so gewählt, dass alle Zähne eines normalen Gebisses beidseitig von den Schneidezähnen bis zu den Backenzähnen überdeckt werden. Die Höhe des Streifens ist so gewählt, dass die oberen und unteren Zähne überdeckt werden. Sowohl in Bezug auf die Länge als auch in Bezug auf die Höhe des Streifens in der Mitte gelegen, befinden sich im Bereich der Schneidezähne durchgehende Öffnungen, die das Atmen ermöglichen. Jeweils seitlich von diesen Atemöffnungen in Richtung Backenzähne befinden sich in Bezug auf die Höhe des Streifens etwa in der Mitte kleine Beissbalken, die senkrecht von dem Streifen abstehen. Die Beissbalken können mit den Backenzähnen gegriffen werden und sollen Zähneknirschen und Muskelverspannungen im Kiefergelenk vorbeugen. Die Beissbalken lassen sich jedoch nicht an das Gebiss des Benutzers anpassen und durch die Atemöffnungen lässt sich nur bei geöffnetem Mund atmen.

Eine orale Apparatur gemäss der WO 00/35369 kann ein Mundschutz oder ein kieferorthopädisches Gerät sein. Die Apparatur weist ein Basiselement mit inneren und äusseren U-förmigen Flanken auf. Dadurch ist ein oberer und unterer Kanal gebildet, in welchem die obere und untere Zahnreihe eines Benutzers aufnehmbar ist. Im Bereich des Basiselements sind Löcher vorgesehen, welche eine Mundatmung bei Anwendung der Apparatur erlauben.

Die orale Apparatur unterstützt den Unterkiefer eines Benutzers in eine anatomisch korrekte Position zu bringen. Insbesondere dass das Basiselement unterschiedliche Dicken aufweist, führt zu einer Entspannung der Nacken- und Kopfmuskulatur. Der Unterkiefer wird durch die orale Apparatur in eine geöffnete und nach vorne verschobene Position gebracht. Diese Kieferposition wirkt sich für einen Sportler auch leistungssteigernd aus.

Zur Herstellung der oralen Apparatur ist ein zweistufiger Spritzgussprozess notwendig. Das Basiselement wird in einer ersten Form aus Polyurethan spritzgegossen. Das Basiselement wird dann in einer zweiten grösseren Form mit EVA überspritzt, da EVA an Polyurethan nicht haftet. Dabei werden die zahnumgebenden Teile ausgeformt. Durch Erwärmung werden zahnumgebenden Teile formbar und lassen sich an das Gebiss des Benutzer anpassen.

### Aufgabe der Erfindung

Aufgabe der vorliegenden Erfindung ist es daher, eine vereinfachte Mundeinlage vorzuschlagen, welche insbesondere günstig in der Herstellung ist und rasch und einfach an das Gebiss eines Benutzers anpassbar ist.

### Beschreibung

Erfindungsgemäss wird die Aufgabe bei einer Vorrichtung gemäss Oberbegriff des Anspruchs 1 dadurch gelöst, dass die wenigstens eine Atemöffnung in den ersten oder zweiten Beissvorsprung integriert ist. Dieses Konstruktionsmerkmal ermöglicht es, Atemöffnung und Beissvorsprung an einer gemeinsamen gewünschten Stelle des Streifens anzuordnen. Auf ein separates Vorsehen wenigstens einer Atemöffnung kann daher verzichtete werden. Dies ist besonders praktisch, wenn der Benutzer selbst die Position des Beissvorsprungs bestimmt. Pro Beissvorsprung mit Atemöffnung braucht dann nur eine Durchgangsöffnung in den Streifen geschnitten werden. Auch ist die gemeinsame Reinigung von Beissvorsprung und Atemöffnung ermöglicht. Bevorzugt ist es, wenn der erste und zweite Beissvorsprung eine Atemöffnung integriert haben. Der Beissvorsprung kann als ein Steg, ein Balken oder ein zylindrischer oder kegelförmiger Vorsprung ausgestaltet sein. Denkbar ist es auch, dass in die Atemöffnung ein Atemluft-Filter integriert ist. Dadurch kann die bei Nasenatmung vorhandene Filterfunktion bei Mundatmung mit der Mundeinlage ebenfalls bereitgestellt werden.

In einer bevorzugten Ausführungsform ist die Atemöffnung durch ein Rohrstück realisiert, dass durch den ersten und/oder zweiten Beissvorsprung hindurchgeführt ist. Das Rohrstück stabilisiert den Beissvorsprung und dient als ein Ansatz, den Beissvorsprung an dem Streifen zu befestigen. Das Rohrstück ist bevorzugt aus einem festen Kunststoff gefertigt, welcher sich leicht reinigen lässt und möglichst keinen Ansatz für Bakterienbildung bietet.

Zweckmässigerweise überragt das Rohrstück an der äusseren Seite des Streifens diesen um wenigstens 10 mm, bevorzugt um 15 mm und besonders bevorzugt um 20 mm. Diese Längenabmessung ermöglicht es, dass der Benutzer auch bei geschlossenen Lippen durch den Mund atmen kann. Falls dies nicht erwünscht ist, können die Rohrstücke jederzeit gekürzt werden.

Neben der Anwendung der Mundeinlage beim Tragen von Zahnspangen bzw. Brackets kann die Mundeinlage auch als Atem-Trainingsgerät eingesetzt werden. Der Mund einer schlafenden Person ist zumeist geschlossen und die schlafende Person atmet durch die Nase. Dies kann das gesamte Atmungssystem während des Schlafs beeinträchtigen. Die Mundeinlage bewirkt, dass genügend Luft durch den Mundatmungsweg während des Schlafs geliefert wird und die Beissnoppen die Kiefermuskulatur fixieren. Während des Schlafs bleibt durch die Mundeinlage der Atmungszyklus des Mund- und Nasenwegs intakt und durch die Fixierung der Kiefermuskulatur wird das gesamte Wirbelsäulensystem entlastet.

Dadurch, dass das Rohrstück in einem bevorzugten Ausführungsbeispiel eine erste, zweite und dritte Kerbe aufweist, sind der Streifen, der Haltering und die Beissnoppe formschlüssig an dem Rohrstück festlegbar. Die Einzelteile bilden eine stabile Einheit, können aber durch Abziehen von dem Rohrstück jederzeit voneinander getrennt werden. Insbesondere für Reinigungszwecke oder wenn eines der Teile kaputt gehen sollte, ist diese Funktion besonders praktisch.

Als vorteilhaft erweist es sich, wenn der Streifen reibschlüssig und/oder formschlüssig auf dem Rohrstück festlegbar ist. Wie im letzten Absatz bereits ausgeführt, lassen sich die Einzelteile durch dieses Konstruktionsmerkmal rasch miteinander verbinden, beispielsweise nachdem der Streifen an die jeweilige Mundgrösse des Benutzers angepasst wurde. Genauso rasch lassen sich die Einzelteile (z.B. für eine Reinigung) auch wieder trennen.

Als vorteilhaft erweist es sich, wenn auf das Rohrstück ein Haltering aufschiebbar ist, welcher im montierten Zustand an der äusseren Seite des Streifens anliegt. Ein Befestigen des Beissvorsprungs gemeinsam mit dem Rohrstück kann daher sehr einfach erfolgen, indem der Haltering auf das vorspringende Rohrstück aufgeschoben wird.

Bevorzugt ist der Haltering reibschlüssig und/oder formschlüssig auf dem Rohrstück festlegbar. Für eine gute Haltefunktion versteht es sich, dass der Haltering aus einem flexiblen Material, beispielsweise aus einem Gummi gefertigt ist. Ferner sollte der Innendurchmesser des Halterings geringfügig kleiner sein als der Aussendurchmesser des Rohrstücks. Um die Haltefunktion noch zu verbessern, ist es denkbar, dass an dem Rohrstück vor der äusseren Seite des Streifens eine umlaufende Einkerbung vorhanden ist. In diese Einkerbung greift der Haltering ein und ist demnach auch formschlüssig an dem Rohstück gehalten.

Gemäß der Erfindung ist der erste und zweite Beissvorsprung eine Beissnoppe, welche im Wesentlichen die Gestalt eines Zylinders aufweist. Die zylindrische Gestalt der Beissnoppe ermöglicht ein komfortables Halten der Mundeinlage mit den Zähnen. Insbesondere wenn die Beissnoppen zwischen 3. und 4. Zähnen der Gebissquadranten angeordnet sind, sind die Beissnoppen für den Benutzer besonders angenehm positioniert. Wie untenstehend beschrieben ist, ist aber auch eine Positionierung der Beissnoppen an jeder anderen Stelle zwischen den Zähnen des Ober- und Unterkiefers möglich.

Zweckmässigerweise weist die Beissnoppe an ihrem der inneren Seite abgewandten Ende eine Frontplatte auf, deren Durchmesser den Durchmesser der restlichen Beissnoppe überragt. Die Frontplatte ermöglicht es dem Benutzer, die Mundeinlage mit den Zähnen noch besser zu fixieren und ein Verrutschen der Mundeinlage weitestgehend zu verhindern.

Mit Vorteil weist die Beissnoppe einen äusseren Teil auf, welcher aus einem weichen, flexiblen Material, insbesondere einem Gummi gefertigt ist. Dieses Material ermöglicht ein angenehmes Halten der Mundeinlage, da sich die Beissnoppen gegenüber dem Druck der Zähne, zwischen welchen sie gehalten sind, verformen.

Gemäß der Erfindung ist die Beissnoppe in den Streifen einklipsbar. Dies kann wie obenstehend beschreiben durch Atemrohr und Haltering ermöglicht sein. Denkbar ist es auch, dass das Rohrstück eine Einkerbung aufweist, welche in eine Durchgangsöffnung an dem Streifen einklipsbar ist.

Dadurch, dass die Aussenkontur des Streifens bevorzugt mit einem Ringwulst versehen ist, besitzt die Mundeinlage keine scharfen Kanten und ist angenehm zu tragen. Für ein komfortables Tragen ist der Ringwulst bevorzugt aus einem weichen flexiblen Material, z.B. Silikon, gefertigt.

Um den Ringwulst und den Übergang zwischen Ringwulst und Streifen gut reinigen zu können, ist es von Vorteil, wenn der Ringwulst am Rand des Streifens umlaufend aufsteckbar ist.

In einer weiteren Ausführungsform sind im Bereich der Aussenkontur Schnittlinien aufgebracht, wodurch der Streifen auf verschiedene Grössen zuschneidbar ist. Dadurch, dass der Streifen zuschneidbar ist, lässt er sich an die individuellen anatomischen Bedürfnisse des Benutzers anpassen. Die Schnittlinien zeigen typische Grössen an dem Streifen an, wodurch sich der Benutzer selbst eine S, M, L oder XL Mundeinlage zuschneiden kann.

Ein weiterer Aspekt der Erfindung bezieht sich auf ein mehrteiliges Set umfassend den Streifen, die Beissnoppen und einen Gummiwulst. Dadurch, dass sich in den Streifen Durchgangsöffnungen schneiden lassen, können die Beissnoppen an jeder Stelle an dem Streifen befestigt werden. Die Schnittlinien für die Durchgangsöffnungen können auf dem Streifen bereits eingezeichnet sein. Dies ist insofern praktisch, da die Durchgangsöffnungen den richtigen Durchmesser für die Beissnoppen besitzen. Bevorzugt sind die Durchgangsöffnungen bzw. die Schnittlinien für die Durchgangsöffnungen symmetrisch zu der Breitenmittellinie und auf der Längenmittellinie des Streifens angeordnet.

Weitere Vorteile und Merkmale ergeben sich aus der nachfolgenden Beschreibung eines Ausführungsbeispiels der Erfindung unter Bezugnahme auf die schematischen Darstellungen. Es zeigen in nicht massstabsgetreuer Darstellung:
- Figur 1:: eine Seitenansicht der erfindungsgemässen Mundeinlage;
- Figur 2:: eine Schnittdarstellung durch die Mundeinlage aus Figur 1 im Bereich einer Beissnoppe;
- Figur 3:: einen Schnitt durch ein Rohrstück und
- Figur 4:: eine perspektivische Ansicht auf die Mundeinlage von schräg oben inkl. zweier Halteringe.

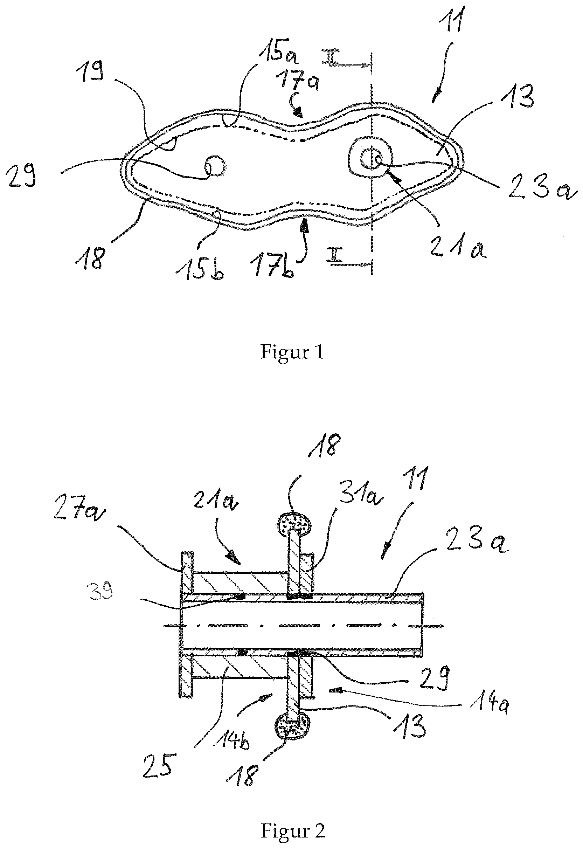

In den Figuren 1, 2 und 4 wird die erfindungsgemässe Mundeinlage gesamthaft mit dem Bezugszeichen 11 bezeichnet. Die Mundeinlage 11 umfasst als ein Basisteil einen Streifen 13 mit einer äusseren Seite 14a, welche beim Tragen den Lippen des Benutzers zugewandt ist, und eine innere Seite 14b, welche den Zähnen des Benutzers zugewandt ist. Die Mundeinlage 11 ist daher im Mundinnenraum zwischen Zähnen und Lippen zu positionieren. Die Länge des Streifens 13 ist so gewählt, dass sie den gesamten Zahnbogen eines Erwachsenen abdeckt. Die Höhe ist so gewählt, dass die Zähne des Oberkiefers und des Unterkiefers abgedeckt werden. Der Streifen ist bevorzugt aus einem flexiblen, abwaschbaren und wasserfesten Folienmaterial gefertigt. Denkbar sind Silikon- oder Gummimaterialien aber auch mit Kunststoff beschichtete Kartonagen o.ä. Nachdem der Streifen biegsam bzw. flexibel sein muss, um sich dem Zahnbogen, bzw. den Lippen eines Benutzers anpassen zu können, besitzt er bevorzugt Schichtdicken von höchstens 1 mm.

Im Bereich der Linie zwischen Kinn und Nase des Benutzers besitzen die Längsseiten 15a,15b des Streifens 13 jeweils eine Einbuchtung 17a,17b, um das Tragen der Mundeinlage 11 komfortabler zu machen. Die Breite des Streifens 13 nimmt in Richtung der Backenzähne wieder ab. Die seitlichen Enden des Streifens 13 sind abgerundet. Zur weiteren Verbesserung des Tragekomforts ist die Aussenkontur des Streifens 13 von einem Ringwulst 18 eingefasst. Der Ringwulst 18 ist innenseitig geschlitzt und lässt sich dadurch einfach entlang der Aussenkontur des Streifens 13 anordnen. Für Reinigungszwecke kann der Ringwulst 18 auch abgenommen werden. Gefertigt ist der Ringwulst 18 aus einem hautschonenden flexiblen Material, beispielsweise Silikon. Damit sich der Ringwulst 18 den Konturen des Streifens 13 besonders gut anpassen kann, kann er auch ein offener Ringwulststreifen sein, oder aus mehreren Ringwulststreifen zusammengesetzt sein. Diese Ringwulststreifen stehen nicht unter Spannung und passen sich den Aussenkonturen bzw. Rundungen des Streifens genau an. Der oder die Ringwulststreifen lassen sich durch Kürzen individuell an die Länge der Aussenkontur des Streifens 13 anpassen.

Es ist vorgesehen, dass der Benutzer der Mundeinlage 11 die Länge und Breite der Mundeinlage 11 an seine Bedürfnisse anpasst, indem er die Seitenränder nach seinen Bedürfnissen abschneidet. Zu diesem Zweck können an dem Streifen 13 im Bereich seiner Aussenkontur eine oder mehrere Schnittlinien 19 eingezeichnet sein. Derartige Schnittlinien 19 sind von Einlegesohlen bekannt, welche an verschiedene Schuhgrössen anpassbar sind.

An dem Streifen 13 lassen sich eine erste und zweite Beissnoppe 21a,21b befestigen. In den Beissnoppen 21a,21b ist jeweils ein Rohrstück 23a, 23b hindurchgeführt. Die Länge des Rohrstücksabschnitts, welcher die äussere Seite des Streifens 13 überragt, ist so gewählt, dass auch bei geschlossenen Lippen durch die Rohrstücke 23a,23b ein- und ausgeatmet werden kann. Wie Figur 2 zeigt, sind die Beissnoppe 21a und das Rohrstück 23a im Wesentlichen senkrecht zu dem Streifen 13 angeordnet. Figur 1 zeigt, dass es bevorzugt ist, wenn die Beissnoppen 21a,21b symmetrisch zur Längen- und Breitenmittellinie angeordnet sind. Im Anschluss an die innere Seite des Streifens 13 ist das Rohrstück 23a, 23b von einem Mittelabschnitt 25a,25b umgeben, welcher aus einem weichen, flexiblen Material, ebenfalls bevorzugt Silikon oder Gummi, besteht. Um das Halten mit den Zähnen zu erleichtern, ist die freie Stirnseite der Beissnoppe 21a,21b mit einer Frontplatte 27a,27b abgeschlossen. Der Durchmesser der Frontplatte 27a,27b ist etwas grösser ausgeführt als der Durchmesser des Mittelabschnitts 25a,25b.

An dem Streifen 13 sind Durchgangsöffnungen 29 vorgesehen, durch welche die Rohrstücke 23a,23b hindurchführbar sind. Dabei entspricht der Innendurchmesser der Durchgangsöffnungen 29 im Wesentlichen dem Aussendurchmesser des Rohrstücks 23a,23b. Die Durchgangsöffnungen 29 können bereits während der Herstellung des Streifens 13 vorgesehen sein, oder sie werden vom Benutzer an den benötigten Stellen ausgeschnitten. Zweckmässigerweise werden die Durchgangsöffnungen 29 an einer Stelle des Streifens 13 ausgeschnitten, sodass sie bei Benutzung zwischen den 3. und 4. Zähnen der Gebissquadranten positioniert sind. Der Benutzer ist jedoch bei der Anordnung der Beissnoppen 23a,23b flexibel. Zur Fixierung der Beissnoppen 23a,23b an dem Streifen 13 sind Halteringe 31a,31b auf die die äussere Seite überragenden Rohrstücke 23a,23b aufschiebbar. Um eine reibschlüssige Halterung der Halteringe 31a,31b zu gewährleisten, sind die Halteringe aus einem flexiblen Material, insbesondere Gummi oder Silikon.

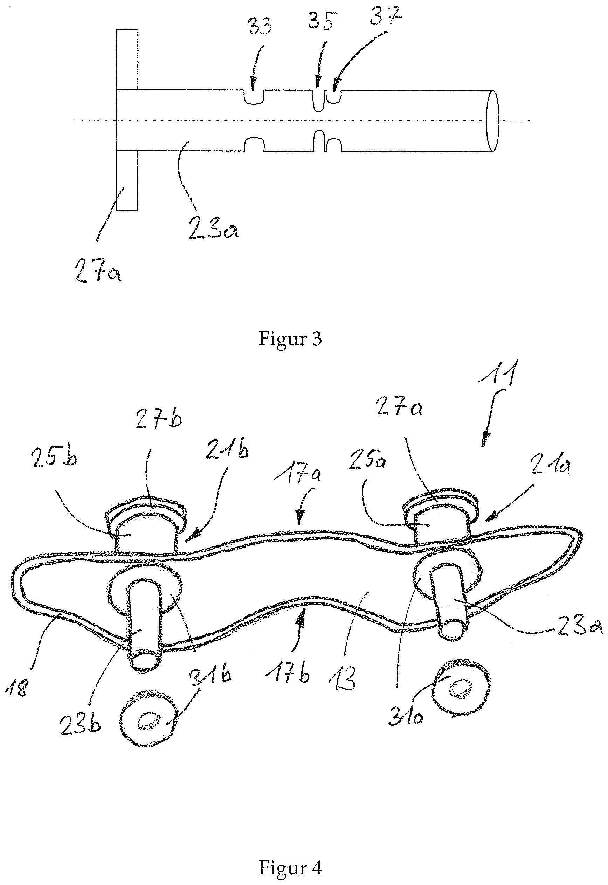

Die Figuren 2 und 3 zeigen, dass die Rohrstücke 23a, 23b jeweils eine erste, zweite und dritte Kerbe 33,35,37 aufweisen. Die erste Kerbe 33 dient der formschlüssigen Halterung des Mittelabschnitts 25a,25b. Der Mittelabschnitt 25a,25b kann dazu Vorsprünge 39 aufweisen, welche in die erste Kerbe 33 einrastbar sind. Die zweite Kerbe 35 dient der formschlüssigen Aufnahme des Streifens 13. Die Durchgangsöffnung 29 des Streifens 13 ist dabei derart bemasst, dass sie in die zweite Kerbe 35 einrasten kann. In ähnlicher Weise ist der Haltering 31a,31b mit der dritten Kerbe 37 verrastbar. Die Kerben 33,35,37 müssen zumindest an einer Stelle an der Oberfläche des Rohrstücks 23a,23b für einen Formschluss vorhanden sein. Es versteht sich, dass die Kerben 22,35,37 auch umlaufend an dem Rohrstück 23a,23b vorhanden sein können. Denkbar ist es auch, auf die dritte Kerbe 37 und den Haltering zu verzichten, da der Streifen 13 in der Kerbe 35 ausreichend fest gehalten ist.

Für Reinigungszwecke sind die Beissnoppen 21a,21b durch Abstreifen der Halteringe 31a,31b rasch von dem Streifen 13 zu trennen und nach der Reinigung auch rasch wieder in den Streifen 13 einsetzbar.

Die Mundeinlage 11 schützt die Innenseite der Lippen vor Brackets zuverlässig und ermöglicht ein Atmen auch, wenn die Lippen geschlossen sind. Ferner entspannt das Tragen der Mundeinlage die Lippen und die Gesichtsmuskulatur. Die integrale Bauform von Rohrstück 23a,23b und Beissnoppen 21a,21b, ermöglicht ein rasches Befestigen und Entfernen dieser Einheit von dem Streifen 13. Die Mundeinlage 11 ist sehr gut an die anatomischen Bedürfnisse des Trägers anpassbar: Zum einen ist die Grösse des Streifens 13 zuschneidbar. Zum anderen sind die Beissnoppen 21a,21b an beliebigen Stellen des Streifens 13 positionierbar, da die Durchgangsöffnungen 29 an beliebigen Stellen des Streifens 13 ausschneidbar sind.

### Legende:

- 11: Mundeinlage
- 13: Streifen
- 14a,14b: Innere/äussere Seite des Streifens 13
- 15a,15b: Längsseiten des Streifens
- 17a,17b: Einbuchtungen
- 18: Ringwulst
- 19: Schnittlinie
- 21a,21b: Erste und zweite Beissnoppe
- 23a,23b: Rohrstück
- 25a,25b: Mittelabschnitt
- 27a,27b: Frontplatte
- 29: Durchgangsöffnung
- 31a,31b: Halteringe
- 33: Erste Kerbe
- 35: Zweite Kerbe
- 37: Dritte Kerbe

## Patentansprüche

1. Mundeinlage (11) zur Positionierung zwischen den Zahnaussenseiten und den Lippen eines Benutzers mit
- einem Streifen (13), eine Länge und Breite sowie eine innere und eine äussere Seite (14a,14b) aufweisend, wobei im Bereich der Längsmitte die Längsseiten eine Einbuchtung (17a,17b) aufweisen,
- wenigstens einer den Streifen (13) durchdringenden Atemöffnung (23) und
- einem ersten Beissvorsprung (21a,), in welchen die Atemöffnung (23) integriert ist,
**dadurch gekennzeichnet,**
- **dass** die Mundeinlage (11) einen zweiten Beissvorsprung (21b) umfasst, wobei der erste und der zweite Beissvorsprung (21a,21b) von der inneren Seite (14b) des Streifens (13) abstehen und
- **dass** der erste und zweite Beissvorsprung Beissnoppen (21a,21b) sind, welche im Wesentlichen die Gestalt eines Zylinders aufweisen und welche in den Streifen (13) einklipsbar sind.

2. Mundeinlage gemäss Anspruch 1, **dadurch gekennzeichnet, dass** die Atemöffnung durch ein Rohrstück (23a,23b) realisiert ist, dass durch den ersten und/oder zweiten Beissvorsprung (21a,21b) hindurchgeführt ist.

3. Mundeinlage gemäss Anspruch 2, **dadurch gekennzeichnet, dass** das Rohrstück (23a,23b) an der äusseren Seite des Streifens (13) diesen um wenigstens 10 mm, bevorzugt um 15 mm und besonders bevorzugt um 20 mm überragt.

4. Mundeinlage gemäss Anspruch 2 oder 3, **dadurch gekennzeichnet, dass** das Rohrstück (23a,23b) eine erste, zweite und dritte Kerbe (33,35,37) aufweist.

5. Mundeinlage gemäss einem der Ansprüche 2 bis 4, **dadurch gekennzeichnet, dass** der Streifen (13) reibschlüssig und/oder formschlüssig auf dem Rohrstück (23a,23b) festlegbar ist.

6. Mundeinlage gemäss einem der Ansprüche 2 bis 5, **dadurch gekennzeichnet, dass** auf das Rohrstück (23a,23b) ein Haltering (31a,31b) aufschiebbar ist, welcher im montierten Zustand an der äusseren Seite des Streifens (13) anliegt.

7. Mundeinlage gemäss Anspruch 6, **dadurch gekennzeichnet, dass** der Haltering (31a,31b) reibschlüssig und/oder formschlüssig auf dem Rohrstück (23a,23b) festlegbar ist.

8. Mundeinlage gemäss Anspruch 1 bis 7, **dadurch gekennzeichnet, dass** die Beissnoppe (21a,21b) an ihrem der inneren Seite (14b) abgewandten Ende eine Frontplatte (27a,27b) aufweist, deren Durchmesser den Durchmesser der restlichen Beissnoppe (21a,21b) überragt.

9. Mundeinlage gemäss einem der Ansprüche 1 bis 8, **dadurch gekennzeichnet, dass** die Beissnoppe (21a,21b) einen äusseren Teil aufweist, welcher aus einem weichen, flexiblen Material, insbesondere einem Gummi gefertigt ist.

10. Mundeinlage gemäss einem der Ansprüche 1 bis 9, **dadurch gekennzeichnet, dass** die Aussenkontur des Streifens (13) mit einem Ringwulst (18) versehen ist.

11. Mundeinlage gemäss Anspruch 10, **dadurch gekennzeichnet, dass** der Ringwulst (18) am Rand des Streifens (13) umlaufend aufsteckbar ist.

12. Mundeinlage gemäss einem der Ansprüche 1 bis 11, **dadurch gekennzeichnet, dass** im Bereich der Aussenkontur Schnittlinien (19) aufgebracht sind, wodurch der Streifen (13) auf verschiedene Grössen zuschneidbar ist.

13. Mundeinlage gemäss einem der Ansprüche 1 bis 12, **dadurch gekennzeichnet, dass** in die Atemöffnung (23) ein Atemluft-Filter integriert ist.

## Claims

1. Oral insert (11) for positioning between the teeth outer sides and the lips of a user with
- a strip (13) that has a length and a width as well as an inner and an outer side (14a, 14b), wherein the long sides have an indentation (17a, 17b) in the region of the longitudinal middle,
- at least one respiratory opening (23) that penetrates the strip (13) and
- a first bite projection (21a) in which the respiratory opening (23) is integrated,
**characterized in**
- **that** the oral insert (11) comprises a second bite projection (21b), wherein the first and the second bite projection (21a, 21b) protrude from the inner side (14b) of the strip (13) and
- **that** the first and the second bite projection are bite nubs (21a, 21b) that have substantially the configuration of a cylinder and that can be clipped into the strip (13).

2. Oral insert according to claim 1, **characterized in that** the respiratory opening is implemented by a tubular piece (23a, 23b) that passes through the first and/or the second bite projection (21a, 21b).

3. Oral insert according to claim 2, **characterized in that** the tubular piece (23a, 23b) on the outer side of the strip (13) projects beyond the strip by at least 10 mm, preferably by 15 mm and particularly preferably by 20 mm.

4. Oral insert according to claim 2 or 3, **characterized in that** the tubular piece (23a, 23b) has a first, a second and a third notch (33, 35, 37).

5. Oral insert according to one of the claims 2 to 4, **characterized in that** the strip (13) can be fixed by friction and/or form-fittingly on the tubular piece (23a, 23b).

6. Oral insert according to one of the claims 2 to 5, **characterized in that** a retaining ring (31a, 31b) can be slid onto the tubular piece (23a, 23b), ring that bears, when mounted, on the outer side of the strip (13).

7. Oral insert according to claim 6, **characterized in that** the retaining ring (31a, 31b) can be fixed by friction and/or form-fittingly on the tubular piece (23a, 23b).

8. Oral insert according to the claims 1 to 7, **characterized in that** the bite nub (21a, 21b) has at its end opposing the inner side (14b), a front plate (27a, 27b), the diameter of which projects over the diameter of the rest of the bite nub (21a, 21b).

9. Oral insert according to one of the claims 1 to 8, **characterized in that** the bite nub (21a, 21b) has an outer part that is made of a soft flexible material, in particular of a rubber.

10. Oral insert according to one of the claims 1 to 9, **characterized in that** the outer contour of the strip (13) is provided with an annular bead (18).

11. Oral insert according to claim 10, **characterized in that** the annular bead (18) can be inserted all around the edge of the strip (13).

## Revendications

1. Insert buccal (11) pour le positionnement entre les faces externes des dents et les lèvres d'un utilisateur avec
- une bande (13) qui présente une longueur et une largeur ainsi qu'un côté interne et un côté externe (14a, 14b), cependant que les longs côtés présentent, dans la zone du milieu longitudinal, une échancrure (17a, 17b),
- au moins une ouverture pour respirer (23) qui traverse la bande (13) et
- une première saillie de morsure (21a) dans laquelle l'ouverture pour respirer (23) est intégrée,
**caractérisé en ce**
- **que** l'insert buccal (11) comprend une seconde saillie de morsure (21b), cependant que la première et la seconde saillie de morsure (21a, 21b) font saillie du côté interne (14b) de la bande (13) et
- **que** la première et la seconde saillie de morsure sont des bossages de morsure (21a, 21b) qui présentent substantiellement la forme d'un cylindre et qui peuvent être enclipsés dans la bande (13).

2. Insert buccal selon la revendication 1, **caractérisé en ce que** l'ouverture pour respirer est réalisée par un élément tubulaire (23a, 23b) qui traverse la première et/ou la seconde saillie de morsure (21a, 21b).

3. Insert buccal selon la revendication 2, **caractérisé en ce que** l'élément tubulaire (23a, 23b) sur le côté externe de la bande (13) dépasse celui-ci d'au moins 10 mm, de préférence de 15 mm et de manière particulièrement préférée de 20 mm.

4. Insert buccal selon la revendication 2 ou 3, **caractérisé en ce que** l'élément tubulaire (23a, 23b) présente une première, une seconde et une troisième entaille (33, 35, 37).

5. Insert buccal selon l'une des revendications 2 à 4, **caractérisé en ce que** la bande (13) peut être fixée par friction et/ou par conjugaison de formes sur l'élément tubulaire (23a, 23b).

6. Insert buccal selon l'une des revendications 2 à 5, **caractérisé en ce qu'**un anneau de retenue (31a, 31b) peut être glissé sur l'élément tubulaire (23a, 23b), anneau qui, à l'état monté, repose sur le côté externe de la bande (13).

7. Insert buccal selon la revendication 6, **caractérisé en ce que** l'anneau de retenue (31a, 31b) peut être fixé par friction et/ou par conjugaison de formes sur l'élément tubulaire (23a, 23b).

8. Insert buccal selon les revendications 1 à 7, **caractérisé en ce que** le bossage de morsure (21a, 21b) présente, à son extrémité opposée au côté interne (14b), une plaque frontale (27a, 27b) dont le diamètre dépasse le diamètre du reste du bossage de morsure (21a, 21b).

9. Insert buccal selon l'une des revendications 1 à 8, **caractérisé en ce que** le bossage de morsure (21a, 21b) présente une partie extérieure qui est fabriquée en une matière souple, flexible, en particulier en un caoutchouc.

10. Insert buccal selon l'une des revendications 1 à 9, **caractérisé en ce que** les contours extérieurs de la bande (13) sont pourvus d'un bourrelet annulaire (18).

11. Insert buccal selon la revendication 10, **caractérisé en ce que** le bourrelet annulaire (18) peut être emboîté à la périphérie sur le bord de la bande (13).
